# EUROPEAN PATENT SPECIFICATION

(11) **EP 3 305 272 B1**
(45) Date of publication and mention of the grant of the patent: **20.04.2022**
(21) Application number: 16800103.0
(22) Date of filing: 26.05.2016
(51) Int. Cl.: A61K 8/27, A61Q 1/00, A61Q 19/00, C10G 9/02, A61K 8/02, A61Q 1/12

(54) **SEBUM ADSORBENT AND COSMETIC COMPRISING SAME**
TALGADSORBENS UND KOSMETIKUM DAMIT
ADSORBANT DE SÉBUM ET PRODUIT COSMÉTIQUE COMPRENANT CELUI-CI

(30) Priority: 28.05.2015 JP 2015108695
(43) Date of publication of application: 11.04.2018
(73) Proprietor: Sakai Chemical Industry Co., Ltd., Sakai-shi, Osaka 590-8502 (JP)
(72) Inventor: HAYASHI, Shintaro, Iwaki-shi Fukushima 971-8183 (JP); ASHIDA, Takuro, Sakai-shi Osaka 590-0985 (JP)
(74) Representative: Isarpatent
(86) International application number: PCT/JP2016/065632
(87) International publication number: WO 2016/190399

(56) References cited:
- EP-A1- 2 824 073
- WO-A1-99/59538
- WO-A1-2013/133412
- JP-A- H 111 411
- JP-A- H07 118 133
- JP-A- S61 257 909
- JP-A- 2002 515 412
- JP-A- 2007 176 822
- KR-A- 20140 141 257
- NORIKO SAITO ET AL: "Hierarchical structures of ZnO spherical particles synthesized solvothermally", SCIENCE AND TECHNOLOGY OF ADVANCED MATERIALS, vol. 12, no. 6, 1 December 2011 (2011-12-01), pages 064707-6, XP055535164, ISSN: 1468-6996, DOI: 10.1088/1468-6996/12/6/064707

## Description

### Technical Field

The present invention relates to a sebum adsorbent and a cosmetic containing the same.

### Background Art

Cosmetics are required to have resistance to perspiration and/or sebum that are secreted by skin and, typically, required not to cause excessive oil on skin and/or smearing of makeup because cosmetics are used by being applied on a skin of human body. To satisfy such requirements, studies have been carried out to avoid smearing of makeup by applying various improvements to powders used in cosmetics.

Examples include methods in which inorganic porous powders such as porous silica and spherical porous magnesium carbonate are blended in cosmetics. However, these powders adsorb moisture on the skin and cause problems of dry skin and/or itchiness of skin because emollient components of the skin become insufficient (JP 2009-137806 A and JP 2006-096706 A). Furthermore, although other organic powders, such as acrylic polymers, have been used as sebum adsorbents, it is hoped that materials having even better sebum-adsorbing performance than the performances of such sebum adsorbents are developed.

Meanwhile, in the field of cosmetics, zinc oxide has been used as a white pigment and/or an ultraviolet blocking material depending on its particle size. For example, it has been known that durability of makeup can be enhanced by allowing microparticulate zinc oxide to react with fatty acid in sebum to fix the sebum, thereby suppressing spread of sebum (JP 08-041379 A). However, such microparticulate zinc oxide has disadvantages of poor texture because squeaky feeling is caused when such microparticulate zinc oxide is blended in cosmetics. Therefore, in a case where zinc oxide particles exhibiting excellent texture and higher sebum-adsorbing performance than zinc oxide particles in the related art can be obtained, the zinc oxide particles are significantly advantageous materials in the field of cosmetics. In particular, zinc oxide particles having a large particle size exhibits excellent impression from use and a certain degree of ultraviolet blocking performance. If the sebum-adsorbing performance of these can be enhanced, such zinc oxide particles can be suitable as a sebum adsorbent having characteristics that are different from characteristics of sebum adsorbents formed from microparticulate zinc oxide and sebum adsorbents other than zinc oxide.

The inventors of the present application filed a patent application related to spherical zinc oxide particles of aggregated lamellar zinc oxide and blending of the particles into cosmetics as WO 2013/133412. However, WO 2013/133412 does not describe sebum adsorbing performance although it describes smooth texture, ultraviolet blocking performance, and the like. Furthermore, although it is mentioned that surface treatment may be performed, specific surface treating agents, surface treatment methods, and effects thereof are not described.

EP 2 824 073 A1 discloses spherical zinc oxide particles consisting of integrated plate-like particles. KR 2014 0141257 A discloses spherical zinc oxide particles comprising aggregated lamellar zinc oxide. WO 99/59538 A1 as well as N. Saito et al., Science and Technology of Advanced Materials (2011), 6, 064707 also disclose spherical zinc oxide particles.

### Summary of Invention

### Technical Problem

The present invention is to provide a sebum adsorbent that achieves high sebum-adsorbing effect while high ultraviolet blocking performance is exhibited and that does not impair the texture, and a cosmetic containing the same.

### Solution to Problem

The present invention relates to a sebum adsorbent containing a spherical zinc oxide of aggregated lamellar zinc oxide having a median size of 0.01 µm or greater but less than 10 µm. The sebum adsorbent contains a spherical zinc oxide of aggregated lamellar zinc oxide that has a median size of 0.01 µm or greater but less than 10 µm and that has been surface-treated with organopolysiloxane and/or triethoxycaprylylsilane so that from 0.5 to 10 parts by weight of the organopolysiloxane and/or the triethoxycaprylylsilane is adhered per 100 parts by weight of the zinc oxide. The organopolysiloxane is preferably dimethyl silicone. The sebum adsorbent is preferably a spherical zinc oxide of aggregated lamellar zinc oxide obtained by a method including a step (1) of neutralizing a zinc salt aqueous solution by an alkali aqueous solution wherein the step (1) is performed in the presence of polyoxyethylene sorbitan monooleate. The present invention also relates to a cosmetic containing the sebum adsorbent described above.

The present invention also relates to a method of adsorbing sebum, the method including applying a spherical zinc oxide of aggregated lamellar zinc oxide having a median size of 0.01 µm or greater but less than 10 µm or a composition containing the spherical zinc oxide of aggregated lamellar zinc oxide on skin. The present invention also relates to use of a spherical zinc oxide of aggregated lamellar zinc oxide having a median size of 0.01 µm or greater but less than 10 µm or a composition containing the spherical zinc oxide of aggregated lamellar zinc oxide for sebum adsorption by application on skin.

### Advantageous Effects of Invention

The cosmetic containing the sebum adsorbent of the present invention not only achieves high ultraviolet blocking performance but also suppresses spreading of sebum by fixing the sebum, and thus is less likely to cause smearing of makeup and achieves excellent impression from use. Furthermore, the sebum-adsorbing performance thereof is significantly superior to the sebum-adsorbing performance of known microparticulate zinc oxide particles in the related art. Furthermore, the sebum-adsorbing performance thereof is higher than the sebum-adsorbing performances of sebum adsorbents of organic powders, such as acrylic polymers.

### Description of Embodiments

The present invention relates to a sebum adsorbent containing a spherical zinc oxide of aggregated lamellar zinc oxide having a median size of 0.01 µm or greater but less than 10 µm so that from 0.5 to 10 parts by weight of the organopolysiloxane and/or the triethoxycaprylylsilane is adhered per 100 parts by weight of the zinc oxide. That is, it was found that zinc oxide having a particular shape and particle size has sebum-adsorbing effect that is superior to the sebum-adsorbing effect of known zinc oxide particles in the related art and thus the present invention has been completed. The zinc oxide having such a sebum-adsorbing effect can be used in various cosmetics and is preferable from the perspective of preventing makeup from being smeared, by adsorbing sebum in the case where the zinc oxide has been blended in makeup cosmetics, such as makeup base and powder makeup.

The spherical zinc oxide of aggregated lamellar zinc oxide of the sebum adsorbent of the present invention has a median size of 0.01 µm or greater but less than 10 µm. The particles having such particle sizes can exhibit excellent performances such as smoothness. The lower limit of the median size is more preferably 0.05 µm or greater, even more preferably 0.1 µm or greater, and particularly preferably 0.2 µm or greater. Note that the median size in the present specification is a size where, when particle sizes of powder were separated into two groups using a particular particle size as a boundary value, the number of the values in the group of larger values becomes equal to the number of the values in the group of smaller values. The median size is a value obtained by the measurement method described in examples below.

The spherical zinc oxide of aggregated lamellar zinc oxide of the sebum adsorbent of the present invention preferably has a BET specific surface area of 5 m²/g or greater. The BET specific surface area in the range described above allows particularly preferable performances to be exhibited as the effect in fixing sebum. That is, it is conceived that, due to the large BET specific surface area, the contact area between sebum and zinc oxide becomes greater, thereby achieving higher sebum-adsorbing performance than the sebum-adsorbing performances of zinc oxide particles in the related art. The BET specific surface area is more preferably 6 m²/g or greater but less than 15 m²/g. Note that the BET specific surface area is a value obtained by the measurement method described in the examples below.

The sebum adsorbent of the present invention is a spherical zinc oxide particle of aggregated lamellar zinc oxides. That is, the zinc oxide particles having such a particular shape readily adsorb sebum due to the shape, thereby achieving the particular effects of the present invention.

The spherical zinc oxide particle of aggregated lamellar zinc oxides are zinc oxide particles in which lamellar zinc oxides are aggregated into a spherical shape thereby making the aggregated particles as a whole into a sphere. Such zinc oxide particles were developed by Patent Document 4 applied by the applicant of the present application.

Although the production method of the spherical zinc oxide particles of aggregated lamellar zinc oxide is not particularly limited, for example, the spherical zinc oxide particles of aggregated lamellar zinc oxide can be obtained by a production method including a step (1) of neutralizing a zinc salt solution by an alkali aqueous solution, the step (1) being performed in the presence of a hydrophilic dispersant. The alkali component contained in the alkali aqueous solution is not particularly limited, and examples thereof include sodium hydroxide, potassium hydroxide, lithium hydroxide, and the like. The used amount of the alkali aqueous solution is preferably in a proportion in which from 2 to 10 mol of alkali component is used per 1 mol of zinc ion.

Furthermore, the hydrophilic dispersant is not particularly limited; however, examples thereof include polycarboxylic acid and salts thereof, alkyl sulfonic acid and salts thereof, alkylbenzene sulfonic acid and salts thereof, naphthalene sulfonic acid and salts thereof, polyether alkyl sulfonic acid and salts thereof, alkylbetaines, polyethers and derivatives thereof, polyether-alkyl ethers, polyoxyalkylene alkenyl phenyl ethers, sorbitan fatty acid esters, polyether sorbitan fatty acid esters, polyether fatty acid esters, glyceryl fatty acid esters, polyether hydrogenated castor oils, polyether alkylamines, polyether-modified silicones, polyglyceryl-modified silicones, polyhydric alcohols, alkyl-modified polyhydric alcohols, and the like. Any of anionic type, cationic type, and nonionic type compounds may be used, but nonionic type compounds are preferred because the nonionic type compounds are less likely to be affected by the water hardness and an electrolyte and can be used with other various surfactants and thus is easy to handle. Furthermore, the HLB value of the hydrophilic dispersant is preferably 10.0 to 20 because the hydrophilic dispersant sufficiently dissolves in water and, in addition to the dispersing effect of the hydrophilic dispersant, the particle growth rate can be fixed by allowing the hydrophilic dispersant to adsorb on a reaction site of the generated particle surface layer. Furthermore, two or more types of the hydrophilic dispersants may be used in combination. Note that, in the present specification, the HLB value is a value obtained according to the Griffin's equation: HLB = (total molecular weight of hydrophilic moieties of the dispersant/molecular weight of the dispersant) × (100/5). Such production method of the spherical zinc oxide particles of aggregated lamellar zinc oxide was developed by Patent Document 4 applied by the applicant of the present application.

Although the reason is not clear, spherical zinc oxide particles of aggregated lamellar zinc oxide having excellent sebum-adsorbing performance can be obtained particularly when polyoxyethylene sorbitan monooleate is used as the hydrophilic dispersant. The use of this as the hydrophilic dispersant is thus particularly preferable.

Furthermore, spherical zinc oxide particles of aggregated lamellar zinc oxide having less odor can be obtained by use of polyoxyethylene sorbitan monolaurate as the hydrophilic dispersant.

The spherical zinc oxide of aggregated lamellar zinc oxide of the sebum adsorbent of the present invention is subjected to a surface treatment in which the surface of the particle is covered by organopolysiloxane and/or triethoxycaprylylsilane. Such a treatment can enhance the sebum-adsorbing performance of the spherical zinc oxide of aggregated lamellar zinc oxide. In particular, covering by organopolysiloxane is preferable from the perspective of superior sebum-adsorbing performance. Furthermore, the organopolysiloxane is preferably dimethyl silicone because the spherical zinc oxide of aggregated lamellar zinc oxide after the surface treatment is not discolored.

In the treatment using the organopolysiloxane or the triethoxycaprylylsilane, from 0.5 to 10 parts by weight of the organopolysiloxane or the triethoxycaprylylsilane is adhered per 100 parts by weight of the zinc oxide. More preferably, the lower limit described above is preferably 1 part by weight and the upper limit described above is preferably 5 parts by weight. When the amount of the organopolysiloxane or the triethoxycaprylylsilane is less than 0.5 part by weight relative to the amount of the zinc oxide, the effect of enhancing sebum-adsorbing performance cannot be exhibited sufficiently. On the other hand, when the amount is greater than 10 parts by weight, the effect of enhancement is saturated and thus such amount is economically disadvantageous.

As described in WO 2013/133412 above, it has been publicly known that the spherical zinc oxide of aggregated lamellar zinc oxide is zinc oxide particles having excellent powder texture, excellent soft focus effect, and high ultraviolet blocking performance. Even when the spherical zinc oxide of aggregated lamellar zinc oxide is treated with the organopolysiloxane or the triethoxycaprylylsilane, sebum-adsorbing performance can be enhanced while the powder texture, the soft focus effect, and the ultraviolet blocking performance are not impaired.

The treatment method using the organopolysiloxane or the triethoxycaprylylsilane is not particularly limited, and examples thereof include a method of dry-mixing using a mixer.

The sebum adsorbent of the present invention preferably has a sebum adsorption amount (mg/g) of 230 mg/g or greater. The sebum adsorption amount within such a range can prevent makeup from being smeared when the sebum adsorbent is blended in cosmetics. The sebum adsorption amount is more preferably 260 mg/g or greater, and even more preferably 270 mg/g or greater. Note that the sebum adsorption amount is a value measured by the method described in detail in the examples.

The sebum adsorbent of the present invention preferably has an average friction coefficient (MIU) of 0.50 to 0.76. The average friction coefficient (MIU) within such a range can provide cosmetics having good slipperiness and smooth texture. Note that the average friction coefficient (MIU) is a value measured by the method described in detail in the examples.

The sebum adsorbent of the present invention preferably has a mean deviation of friction coefficient (MMD) of 0.0080 to 0.0200. The mean deviation of friction coefficient (MMD) within such a range can provide cosmetics having good slipperiness and smooth texture. Note that the mean deviation of friction coefficient (MMD) is a value measured by the method described in detail in the examples.

The sebum adsorbent of the present invention preferably has a total light transmittance of 40% or less. The total light transmittance within such a range can provide zinc oxide particles having excellent ultraviolet blocking effect. Note that the total light transmittance is a value measured by the method described in detail in the examples.

The present invention also relates to a cosmetic containing the sebum adsorbent formed from the zinc oxide composed of aggregated lamellar zinc oxides described above. The compounded amount of the spherical zinc oxide of aggregated lamellar zinc oxide in the cosmetic of the present invention is from 0.5 to 90 wt.%, and particularly preferably from 1 to 85 wt.%, in the entire composition. When the compounded amount is less than 0.5 wt.%, the sebum-adsorbing effect is low, and when the compounded amount is greater than 90 wt.%, texture becomes poor due to excessive adsorption of sebum.

In the cosmetic containing the sebum adsorbent of the present invention, any aqueous component or oily component that can be used in the field of cosmetics can be used in combination in addition to the sebum adsorbent described above. The aqueous component and oily component described above are not particularly limited. Examples thereof may include those containing components such as oils, surfactants, moisturizers, higher alcohols, sequestrants, natural and synthetic polymers, water-soluble and oil-soluble polymers, ultraviolet blocking agents, various extracts, inorganic and organic pigments, inorganic and organic clay minerals and other powders, coloring materials such as organic dyes, preservatives, antioxidants, dyes, thickeners, pH adjusters, perfumes, cooling-sensation agents, antiperspirants, disinfectants, and skin activators. Specifically, a desired cosmetic can be produced in the usual manner using any one or two or more types of the ingredients listed below. The compounded amounts of these ingredients are not particularly limited as long as the compounded amounts are in the range that do not impair the effect of the present invention.

The oil is not particularly limited. Examples thereof may include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, arachis oil, tea seed oil, kaya oil, rice bran oil, Chinese tung oil, Japanese tung oil, jojoba oil, germ oil, triglycerol, glycerol trioctanoate, glycerol triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol ester, POE hydrogenated lanolin alcohol ether, liquid paraffin, ozokerite, pristane, paraffin, ceresin, squalene, vaseline, microcrystalline wax, silicone oil, and the like.

The lipophilic nonionic surfactant is not particularly limited. Examples thereof may include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acid esters such as glycerol mono-cottonseed oil fatty acid, glycerol monoerucate, glycerol sesquioleate, glycerol monostearate, α,α'-glycerol oleate pyroglutamate, and glycerol monostearate malate; propylene glycol fatty acid esters such as propylene glycol monostearate; hydrogenated castor oil derivatives; glycerol alkyl ethers; polyether-modified silicones; polyglycerol-modified silicones; amino-modified silicones; and the like.

The hydrophilic nonionic surfactant is not particularly limited. Examples thereof may include POE sorbitan fatty acid esters such as POE sorbitan monooleate, POE sorbitan monostearate, POE sorbitan monooleate, and POE sorbitan tetraoleate; POE sorbitol fatty acid esters such as POE sorbitol monolaurate, POE sorbitol monooleate, POE sorbitol pentaoleate, and POE sorbitol monostearate; POE glycerin fatty acid esters such as POE glycerin monostearate, POE glycerin monoisostearate, and POE glycerin triisostearate; POE fatty acid esters such as POE monooleate, POE distearate, POE monodioleate, and ethylene glycol distearate; POE alkyl ethers such as POE lauryl ether, POE oleyl ether, POE stearyl ether, POE behenyl ether, POE 2-octyldodecyl ether, and POE cholestanol ether; POE alkyl phenyl ethers such as POE octyl phenyl ether, POE nonyl phenyl ether, and POE dinonyl phenyl ether; Pluaronic types such as Pluronic; POE/POP alkyl ethers such as POE/POP cetyl ether, POE/POP 2-decyltetradecyl ether, POE/POP monobutyl ether, POE/POP hydrogenated lanolin, and POE/POP glycerin ether; tetra-POE/tetra-POP ethylenediamine condensation products such as Tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleic acid; POE beeswax/lanolin derivatives such as POE sorbitol beeswax; alkanolamides such as coconut oil fatty acid diethanolamide, lauric acid monoethanolamide, and fatty acid isopropanol amide; POE propylene glycol fatty acid esters; POE alkylamines; POE fatty acid amides; sucrose fatty acid esters; POE nonylphenyl formaldehyde condensation products; alkyl ethoxy dimethylamine oxides; trioleyl phosphoric acid; and the like.

Other surfactants may be compounded in the range that does not cause any problems in stability and skin irritation. Examples thereof include anionic surfactants such as fatty acid soaps, higher-alkyl sulfuric ester salts, POE triethanolamine lauryl sulfate, and alkyl ether sulfuric ester salts; cationic surfactants such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants such as imidazoline amphoteric surfactants and betaine surfactants.

The moisturizer is not particularly limited. Examples thereof may include xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, melilot extract, and the like.

The higher alcohol is not particularly limited. Examples thereof may include linear alcohols such as lauryl alcohol, cetyl alcohol, stearyl alcohol, behenyl alcohol, myristyl alcohol, oleyl alcohol, and cetostearyl alcohol; branched alcohols such as monostearyl glycerol ether (batyl alcohol), 2-decyltetradecynol, lanolin alcohol, cholesterol, phytosterol, hexyldodecanol, isostearyl alcohol, and octyldodecanol; and the like.

The sequestrant is not particularly limited. Examples thereof may include 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, edetic acid, and the like.

The natural water-soluble polymer is not particularly limited. Examples thereof may include plant-derived polymers such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers such as xanthan gum, dextran, succinoglucan, and pullulan; and animal-derived polymers such as collagen, casein, albumin, and gelatin.

The semisynthetic water-soluble polymer is not particularly limited. Examples thereof may include starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; alginate polymers such as sodium alginate and propylene glycol alginate; and the like.

The synthetic water-soluble polymer is not particularly limited. Examples thereof may include vinyl polymers such as polyvinyl alcohol, polyvinyl methyl ether, and polyvinyl pyrrolidone; polyoxyethylene polymers such as polyethylene glycol 20000, polyethylene glycol 40000, and polyethylene glycol 60000; copolymers such as polyoxyethylene-polyoxypropylene copolymers; acrylic polymers such as sodium polyacrylate, polyethylacrylate, and polyacrylamide; polyethyleneimine; cationic polymers; and the like.

The inorganic water-soluble polymer is not particularly limited. Examples thereof may include bentonite, magnesium aluminum silicate (Veegum), laponite, hectorite, silicic anhydride, and the like.

The ultraviolet blocking agent is not particularly limited. Examples thereof may include benzoic acid-based ultraviolet blocking agents such as paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet blocking agents such as homomenthyl-N-acetyl anthranilate; salicylic acid-based ultraviolet blocking agents such as amyl salicylate, menthyl salicylate, homomenthyl salicylate, octyl salicylate, phenyl salicylate, benzyl salicylate, and p-isopropanol phenyl salicylate; cinnamic acid-based ultraviolet blocking agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glycerylmono-2-ethylhexanoyl-diparamethoxy cinnamate; benzophenone-based ultraviolet blocking agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t-butyldibenzoylmethane, 5-(3,3-dimethyl-2-norbornylidene)-3-pentan-2-one, and the like.

Various kinds of extracts are not particularly limited. Examples thereof may include Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, licorice extract, and the like.

Examples of the various kinds of powders may include bright coloring pigments such as red oxide, yellow iron oxide, black iron oxide, mica titanium, iron oxide-coated mica titanium, and titanium oxide-coated glass flakes, inorganic powders such as those of mica, talc, kaolin, sericite, titanium dioxide, silica, barium sulfate, and calcium carbonate, organic powders such as polyethylene powder, nylon powder, crosslinked polystyrene, cellulose powder, and silicone powder, and the like. Preferably, for enhancing sensory characteristics and makeup retainability, part or all of the powder component may be subjected to a hydrophobization treatment by a publicly known method using a substance such as a silicone, a fluorine compound, a metallic soap, an oily agent, or an acyl glutamic acid salt, for use. Other zinc oxide particles that do not fall under the present invention may be mixed and used.

The technique of the present invention as described above may be understood as a method of adsorbing sebum by applying a spherical zinc oxide of aggregated lamellar zinc oxide having a median size of 0.01 µm or greater but less than 10 µm and surface-treated with organopolysiloxane and/or triethoxycaprylylsilane or a composition containing said surface-treated spherical zinc oxide of aggregated lamellar zinc oxide on skin. The present invention may be also understood as the use of a spherical zinc oxide of aggregated lamellar zinc oxide having a median size of 0.01 µm or greater but less than 10 µm and surface-treated with organopolysiloxane and/or triethoxycaprylylsilane or a composition comprising said surface-treated spherical zinc oxide of aggregated lamellar zinc oxide for sebum adsorption by application on skin.

### Examples

The present invention will be described in detail hereinafter with reference to examples, but the present invention is not limited to these examples alone. Note that, unless otherwise specified, "%" refers to "wt.%" in the following descriptions.

### Example 1

In water, 32 g of zinc acetate dihydrate (manufactured by Kishida Chemical Co., Ltd.; purity: 98%) was dissolved to prepare 116 mL of zinc acetate aqueous solution having a zinc acetate dihydrate concentration of 1.26 mol/L. In 758 mL of sodium hydroxide aqueous solution that was prepared by dissolving 31.3 g of sodium hydroxide (manufactured by Kishida Chemical Co., Ltd.; purity: 98%) in water and that had a sodium hydroxide concentration of 1.0 mol/L, 2.125 g of TW-O120V (manufactured by Kao Corporation; polyoxyethylene sorbitan monooleate; HLB value: 14.9) was added and sufficiently mixed. The sodium hydroxide aqueous solution was then stirred at a rotation speed of 300 rpm using a stirring machine, and the zinc acetate aqueous solution was added and mixed in 10 seconds, and then the mixture was stirred for 30 minutes to allow the reaction to proceed. After completion of the reaction, filtration, water-washing, and drying were performed to obtain spherical zinc oxide particles of aggregated lamellar zinc oxide having a median size of 1.28 µm.

### Example 2

To 100 parts by weight of the spherical zinc oxide particles of aggregated lamellar zinc oxide obtained in Example 1, 2 parts by weight of dimethyl silicone oil (KF-96A-100cs, manufactured by Shin-Etsu Chemical Co., Ltd.) was added and mixed using a sample mill, and a dry processing was performed.

### Example 3

In water, 32 g of zinc acetate dihydrate (manufactured by Kishida Chemical Co., Ltd.; purity: 98%) was dissolved to prepare 116 mL of zinc acetate aqueous solution having a zinc acetate dihydrate concentration of 1.26 mol/L. In 758 mL of sodium hydroxide aqueous solution that was prepared by dissolving 31.3 g of sodium hydroxide (manufactured by Kishida Chemical Co., Ltd.; purity: 98%) in water and that had a sodium hydroxide concentration of 1.0 mol/L, 2.125 g of TW-L120 (manufactured by Kao Corporation; polyoxyethylene sorbitan monolaurate; HLB value: 16.7) was added and sufficiently mixed. The sodium hydroxide aqueous solution was then stirred at a rotation speed of 300 rpm using a stirring machine, and the zinc acetate aqueous solution was added and mixed in 10 seconds, and then the mixture was stirred for 30 minutes to allow the reaction to proceed. After completion of the reaction, filtration, water-washing, and drying were performed to obtain spherical zinc oxide particles of aggregated lamellar zinc oxide having a median size of 1.16 µm. The spherical zinc oxide particles of aggregated lamellar zinc oxide of Example 3 obtained as described above had less odor compared to the zinc oxide particles of Example 1.

### Example 4

To 100 parts by weight of the spherical zinc oxide particles of aggregated lamellar zinc oxide obtained in Example 3, 2 parts by weight of dimethyl silicone oil (KF-96A-100cs, manufactured by Shin-Etsu Chemical Co., Ltd.) was added and mixed using a sample mill, and a dry processing was performed.

### Example 5

To 100 parts by weight of the spherical zinc oxide particles of aggregated lamellar zinc oxide obtained in Example 1, 2 parts by weight of triethoxycaprylylsilane (AES-3083, manufactured by Shin-Etsu Chemical Co., Ltd.) was added and mixed using a sample mill, and a dry processing was performed.

### Reference Example

To 100 parts by weight of the spherical zinc oxide particles of aggregated lamellar zinc oxide obtained in Example 1, 2 parts by weight of hydrogen dimethicone (KF-9901, manufactured by Shin-Etsu Chemical Co., Ltd.) was added and mixed using a sample mill, and then dried at 120°C for 12 hours. The obtained hydrogen dimethicone-treated spherical zinc oxide particles of aggregated lamellar zinc oxide were turned yellow.

The sebum adsorption amount, median size, specific surface area, average friction coefficient (MIU), mean deviation of friction coefficient (MMD), and total light transmittance of Examples 1 to 5 were measured and shown in Table 1. Furthermore, commercially available microparticulate zinc oxide particles (FINEX-50, manufactured by Sakai Chemical Industry Co., Ltd), commercially available zinc oxide particles (zinc oxide No. 1 (JIS), manufactured by Sakai Chemical Industry Co., Ltd), and acrylic beads typically used as sebum adsorbents of cosmetics (JURYMER MB-1XJ, manufactured by Toagosei Co., Ltd.) as Comparative Examples 1 to 3 were measured in the same manner and shown in Table 1. Note that the measurement methods were methods described below.

**[Table 1]**

| | Example 1 | Example 2 | Example 3 | Example 4 | Example 5 | Comparative Example 1 | Comparative Example 2 | Comparative Example 3 |
|---|---|---|---|---|---|---|---|---|
| | Spherical zinc oxide of aggregated lamellar zinc oxide (without surface treatment) | Spherical zinc oxide of aggregated lamellar zinc oxide (with surface treatment) | Spherical zinc oxide of aggregated lamellar zinc oxide (without surface treatment) | Spherical zinc oxide of aggregated lamellar zinc oxide (with surface treatment) | Spherical zinc oxide of aggregated lamellar zinc oxide (with surface treatment) | Microparticulat e zinc oxide (FINEX-50) | Indefinite-shaped zinc oxide (zinc oxide No. 1 (JIS)) | Acrylic polymer |
| Sebum adsorption amount (mg/g) | 285 | 302 | 230 | 266 | 292 | 198 | 116 | 253 |
| Median size (µm) | 1.28 | 1.23 | 1.16 | 1.16 | 1.42 | 1.55 | 0.70 | 5.52 |
| Specific surface area (m²/g) | 12 | 8.7 | 9.9 | 6.0 | 8.1 | 50 | 3.3 | 1.2 |
| Average friction coefficient (MIU) | 0.70 | 0.68 | 0.75 | 0.71 | 0.76 | 1.06 | 0.83 | 0.53 |
| Mean deviation of friction coefficient (MMD) | 0.0106 | 0.0127 | 0.0146 | 0.0160 | 0.0119 | 0.0558 | 0.0304 | 0.0113 |
| Total light transmittance (%) | 31 | 31 | 31 | 31 | 30 | 31 | 35 | 93 |

Using the powders obtained in Examples and Comparative Examples 1 and 2, baby powders were produced as Examples 6 to 11 and Comparative Examples 4 to 6, and the physical properties were measured in the same manner and shown in Table 2.

### Example 6

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of spherical zinc oxide of aggregated lamellar zinc oxide obtained in Example 1 using a sample mill (compounding ratio was talc:corn starch:zinc oxide = 87:5:8).

### Example 7

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of spherical zinc oxide of aggregated lamellar zinc oxide obtained in Example 2 using a sample mill (compounding ratio was talc:corn starch:zinc oxide = 87:5:8).

### Example 8

A baby powder was obtained by mixing 28.2 g of talc (JA-80R, manufactured by Asada Milling Co., Ltd.), 1.5 g of corn starch (DRY-FLO PURE, manufactured by Akzo Nobel), and 0.3 g of spherical zinc oxide of aggregated lamellar zinc oxide obtained in Example 2 using a sample mill (compounding ratio was talc:corn starch:zinc oxide = 94:5:1).

### Example 9

A baby powder was obtained by mixing 6 g of talc (JA-80R, manufactured by Asada Milling Co., Ltd.) and 24 g of spherical zinc oxide of aggregated lamellar zinc oxide obtained in Example 2 using a sample mill (compounding ratio was talc:zinc oxide = 20:80).

### Example 10

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of spherical zinc oxide of aggregated lamellar zinc oxide obtained in Example 3 using a sample mill (compounding ratio was talc:corn starch:zinc oxide = 87:5:8).

### Example 11

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of spherical zinc oxide of aggregated lamellar zinc oxide obtained in Example 4 using a sample mill (compounding ratio was talc:corn starch:zinc oxide = 87:5:8).

### Comparative Example 4

A baby powder was obtained by mixing 28.5 g of talc (JA-80R, manufactured by Asada Milling Co., Ltd.) and 1.5 g of corn starch (DRY-FLO PURE, manufactured by Akzo Nobel) using a sample mill (compounding ratio was talc:corn starch = 95:5).

### Comparative Example 5

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of zinc oxide of Comparative Example 1 using a sample mill. (compounding ratio was talc:corn starch:zinc oxide = 87:5:8)

### Comparative Example 6

A baby powder was obtained by mixing 26.1 g of talc (JA-80R, manufactured by Asada Milling Co., Ltd.), 1.5 g of corn starch (DRY-FLO PURE, manufactured by Akzo Nobel), and 2.4 g of zinc oxide of Comparative Example 2 using a sample mill. (compounding ratio was talc:corn starch:zinc oxide = 87:5:8)

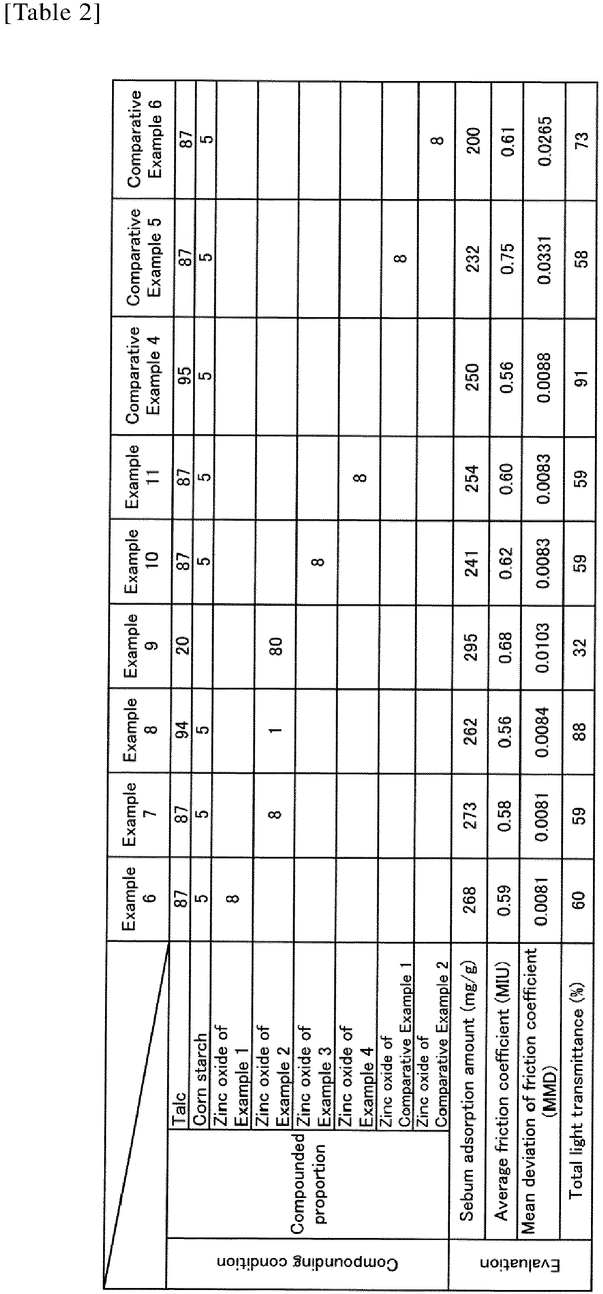

### Measurement of sebum adsorption amount

### (1) Preparation of artificial sebum and preparation of calibration curve

Using a homodisper, 20% of oleic acid, 40% of olive oil, and 40% of squalene were stirred for 10 minutes to prepare artificial sebum. This artificial sebum was used for preparation in a manner that the concentration of the artificial sebum was 1%, 5%, 10%, 20%, and 50% in isopropyl alcohol. A drop of each of artificial sebum which was diluted with isopropyl alcohol to the concentrations was placed on an aluminum foil and then transferred using a hydraulic press. The transferred portion was measured using an infrared spectrophotometer NICOLET iS10 (manufactured by Thermo Fisher Scientific Inc.) to calculate the peak area of asymmetric stretching of C-H in 2945 to 2875 cm⁻¹, thereby preparing a calibration curve of the concentrations of artificial sebum and the peak areas.

### (2) Measurement of sebum amount adsorbed on powder

In a centrifuge tube, 2.5 g of artificial sebum and 7 g of isopropyl alcohol were charged and mixed with 0.5 g of each of the powders of Examples 1 to 5 and Comparative Examples 1 to 3 (or the obtained baby powder in the case of Examples 6 to 11 and Comparative Examples 4 to 6) and left for 30 minutes. After being left, the cake and the solvent were separated using a high-performance centrifuge Avanti HP-26XP (manufactured by Beckman Coulter) at 8000 rpm for 30 minutes, and the solvent was charged in a screw tube. Thereafter, a drop of the solvent was placed on an aluminum foil and then transferred using a hydraulic press. The transferred portion was measured using an infrared spectrophotometer NICOLET iS10 (manufactured by Thermo Fisher Scientific Inc.) to calculate the peak area of asymmetric stretching of C-H in 2945 to 2875 cm⁻¹. From the peak area, the artificial sebum amount in the solvent was calculated backwards using the calibration curve produced in (1). The sebum adsorption amount (mg/g) was determined by calculating the proportion (%) of the artificial sebum adsorbed on the powder from the difference between the concentration of the solvent after the centrifugal separation and the concentration of the mixed solution of artificial sebum and isopropyl alcohol before the mixing of the powder of example or comparative example as a reference. A greater value of the sebum adsorption amount (mg/g) indicates that spreading of sebum can be suppressed by reacting with the sebum and fixing the sebum, and thus is effective for preventing smearing of makeup.

### Median size

The median size was measured using a laser diffraction/scattering particle size distribution measuring device LA-750 (manufactured by HORIBA, Ltd.).

### Specific surface area

The specific surface area (m²/g) was measured using an automatic BET specific surface area measurement device Macsorb (manufactured by Mountech Co., Ltd.)

### Average friction coefficient (MIU)

The average friction coefficient (MIU) in Tables 1 and 2 was a value determined by measuring the powders obtained in the examples and comparative examples with the use of KES-SE friction tester (manufactured by Kato Tech Co., Ltd.). A 25-mm-wide double-stick tape was stuck on a slide glass, and a powder was placed thereon and spread by a makeup puff. Then, the average friction coefficient (MIU) of the obtained sample was measured with the use of KES-SE friction tester (manufactured by Kato Tech Co., Ltd.). The measurement was performed at a friction measurement load of 25 gf, at a surface measurement sample moving speed of 1 mm/sec, and a measurement distance range of 20 mm. As a sensor, a silicone contact piece (a friction piece of silicone rubber provided with protrusions and recessions, assumed as a human finger) was used. A smaller value of the average friction coefficient (MIU) indicates superior smoothness.

### Mean deviation of friction coefficient (MMD)

The mean deviation of friction coefficient (MMD) in Tables 1 and 2 was a value determined by measuring the powders of the examples or comparative examples with the use of KES-SE friction tester (manufactured by Kato Tech Co., Ltd.). A 25-mm-wide double-stick tape was stuck on a slide glass, and a powder was placed thereon and spread by a makeup puff. Then, the mean deviation of friction coefficient (MMD) of the obtained sample was measured with the use of KES-SE friction tester (manufactured by Kato Tech Co., Ltd.). The measurement was performed at a friction measurement load of 25 gf, at a surface measurement sample moving speed of 1 mm/sec, and a measurement distance range of 20 mm. As a sensor, a silicone contact piece (a friction piece of silicone rubber provided with protrusions and recessions, assumed as a human finger) was used. A smaller value of the mean deviation of friction coefficient (MMD) indicates less roughness and superior smoothness.

### Production of coating film

0.1 g of the powders of the examples or comparative examples and 0.9 g of vaseline (manufactured by Wako Pure Chemical Industries, Ltd.) were kneaded and rotated 50 times using an automatic hoover muller for laboratory (manufactured by Toyo Seiki Seisaku-sho, Ltd.) at a rotation speed of 100 rpm and a load of 10 (lb). Some drops of the kneaded product were placed on a glass plate, and a coating film was produced by a bar coater (No. 579 ROD No. 30, manufactured by Yasuda Seiki Seisakusho, Ltd.). Using the coating film immediately after the production, the total light transmittance (%) at a wavelength of 350 nm was measured. A smaller value of the total light transmittance (%) indicates higher ultraviolet blocking effect against ultraviolet light having a wavelength of UVA.

From the results of Table 1, the following was confirmed. It was confirmed that the spherical zinc oxide particles of aggregated lamellar zinc oxide of the present invention exhibited better sebum-adsorbing performance and better texture than those of the microparticulate zinc oxide having the large specific surface area (Comparative Example 1) and the indefinite-shaped zinc oxide having approximately the same median size (Comparative Example 2). Furthermore, in particular, it was confirmed that the spherical zinc oxide particles of aggregated lamellar zinc oxide of Examples 1, 2, 4, and 5 of the present invention exhibited better sebum-adsorbing performance and better ultraviolet blocking performance than those of acrylic beads typically used as sebum adsorbents and/or texture improving agents (Comparative Example 3). Furthermore, it was confirmed that, when Example 1 and Example 3 are compared, the spherical zinc oxide particles of aggregated lamellar zinc oxide, in which polyoxyethylene sorbitan monooleate was used as the hydrophilic dispersant in the production step, of the present invention exhibited even higher sebum-adsorbing performance. Furthermore, when Example 1 and Example 2, Example 1 and Example 5, and Example 3 and Example 4 are compared, it was confirmed that the spherical zinc oxide particles of aggregated lamellar zinc oxide of the present invention can enhance sebum-adsorbing performance by being subjected to the surface treatment using organopolysiloxane or triethoxycaprylylsilane. In particular, it was confirmed that the surface treatment with organopolysiloxane can further enhance the sebum-adsorbing performance.

Therefore, it was found that the sebum adsorbent containing the spherical zinc oxide particles of aggregated lamellar zinc oxide of the present invention is a material having high sebum-adsorbing performance and ultraviolet blocking performance and having excellent texture. Furthermore, from the results of Table 2, it was confirmed that the baby powder containing the sebum adsorbent containing the spherical zinc oxide particles of aggregated lamellar zinc oxide of the present invention exhibited higher sebum-adsorbing performance and better ultraviolet blocking performance than those of the baby powder which did not contain the sebum adsorbent of the present invention (Comparative Example 4). Furthermore, it was confirmed that the baby powder containing the sebum adsorbent of the present invention exhibited better sebum-adsorbing performance and better texture than those of the baby powders containing conventional zinc oxide (Comparative Examples 5 and 6). Therefore, the cosmetic containing the sebum adsorbent of the present invention exhibits excellent texture and can have high sebum-adsorbing performance and ultraviolet blocking performance.

### Industrial Applicability

The sebum adsorbent of the present invention is effective to suppress smearing of makeup and can be suitably used as a raw material for cosmetics having high ultraviolet blocking performance without impairing texture.

## Claims

1. A sebum adsorbent comprising a spherical zinc oxide of aggregated lamellar zinc oxide having a median size of 0.01 µm or greater but less than 10 µm and surface-treated with organopolysiloxane and/or triethoxycaprylylsilane so that from 0.5 to 10 parts by weight of the organopolysiloxane and/or the triethoxycaprylylsilane is adhered per 100 parts by weight of the zinc oxide.

2. The sebum adsorbent according to claim 1, wherein
the organopolysiloxane is dimethyl silicone.

3. The sebum adsorbent according to claim 1 or 2, wherein
the spherical zinc oxide of aggregated lamellar zinc oxide is obtained by a method comprising a step (1) of neutralizing a zinc salt aqueous solution by an alkali aqueous solution, and
the step (1) is performed in the presence of polyoxyethylene sorbitan monooleate.

4. A cosmetic comprising the sebum adsorbent described in claim 1, 2 or 3.

5. A method of adsorbing sebum, the method comprising applying a spherical zinc oxide of aggregated lamellar zinc oxide having a median size of 0.01 µm or greater but less than 10 µm and surface-treated with organopolysiloxane and/or triethoxycaprylylsilane, so that from 0.5 to 10 parts by weight of the organopolysiloxane and/or the triethoxycaprylylsilane is adhered per 100 parts by weight of the zinc oxide, or a composition comprising said surface-treated spherical zinc oxide of aggregated lamellar zinc oxide on skin.

6. Use of a spherical zinc oxide of aggregated lamellar zinc oxide having a median size of 0.01 µm or greater but less than 10 µm and surface-treated with organopolysiloxane and/or triethoxycaprylylsilane, so that from 0.5 to 10 parts by weight of the organopolysiloxane and/or the triethoxycaprylylsilane is adhered per 100 parts by weight of the zinc oxide, or of a composition comprising said surface-treated spherical zinc oxide of aggregated lamellar zinc oxide for sebum adsorption by application on skin.

## Patentansprüche

1. Talgadsorbens, enthaltend ein kugelförmiges Zinkoxid von aggregiertem lamellenförmigem Zinkoxid, das eine Mediangröße von 0,01 µm oder größer, aber kleiner als 10 µm aufweist und mit Organopolysiloxan und/oder Triethoxycaprylylsilan oberflächenbehandelt ist, so dass von 0,5 bis 10 Gewichtsteilen des Organopolysiloxans und/oder des Triethoxycaprylylsilan pro 100 Gewichtsteile des Zinkoxids angehaftet sind.

2. Talgadsorbens nach Anspruch 1, wobei das Organopolysiloxan Dimethylsilikon ist.

3. Talgadsorbens nach Anspruch 1 oder 2, wobei
das kugelförmige Zinkoxid von aggregiertem lamellenförmigem Zinkoxid mit einem Verfahren, umfassend einen Schritt (1) des Neutralisierens einer wässrigen Zinksalzlösung mit einer wässrigen Alkalilösung, erhalten wird und
der Schritt (1) in Gegenwart von Polyoxyethylensorbitanmonooleat durchgeführt wird.

4. Kosmetikum, enthaltend das in Anspruch 1, 2 oder 3 beschriebene Talgadsorbens.

5. Verfahren zum Adsorbieren von Talg, wobei das Verfahren umfasst: Auftragen eines kugelförmigen Zinkoxids von aggregiertem lamellenförmigem Zinkoxid, das eine Mediangröße von 0,01 µm oder größer, aber kleiner als 10 µm aufweist und mit Organopolysiloxan und/oder Triethoxycaprylylsilan oberflächenbehandelt ist, so dass von 0,5 bis 10 Gewichtsteilen des Organopolysiloxans und/oder des Triethoxycaprylylsilans pro 100 Gewichtsteile des Zinkoxids angehaftet sind, oder einer Zusammensetzung, enthaltend das oberflächenbehandelte kugelförmige Zinkoxid von aggregiertem lamellenförmigem Zinkoxid, auf die Haut.

6. Verwendung eines kugelförmigen Zinkoxids von aggregiertem lamellenförmigem Zinkoxid, das eine Mediangröße von 0,01 µm oder größer, aber kleiner als 10 µm aufweist und mit Organopolysiloxan und/oder Triethoxycaprylylsilan oberflächenbehandelt ist, so dass von 0,5 bis 10 Gewichtsteilen des Organopolysiloxans und/oder des Triethoxycaprylylsilans pro 100 Gewichtsteile des Zinkoxids angehaftet sind, oder einer Zusammensetzung, enthaltend das oberflächenbehandelte kugelförmige Zinkoxid von aggregiertem lamellenförmigem Zinkoxid, zur Adsorption von Talg durch Auftragen auf die Haut.

## Revendications

1. Adsorbant de sébum comprenant un oxyde de zinc sphérique d'oxyde de zinc lamellaire agrégé ayant une taille médiane de 0,01 µm ou plus mais de moins de 10 µm et traité en surface avec l'organopolysiloxane et/ou le triéthoxycaprylylsilane de sorte que de 0,5 à 10 parties en poids de l'organopolysiloxane et/ou du triéthoxycaprylylsilane est collé par 100 parties en poids de l'oxyde de zinc.

2. Adsorbant de sébum selon la revendication 1, dans lequel l'organopolysiloxane est la diméthyl silicone.

3. Adsorbant de sébum selon la revendication 1 ou 2, dans lequel l'oxyde de zinc sphérique de l'oxyde de zinc lamellaire agrégé est obtenu par un procédé comprenant une étape (1) de neutralisation d'une solution aqueuse de sel de zinc par une solution aqueuse alcaline, et
l'étape (1) est effectuée en présence de monooléate de polyoxyéthylène sorbitan.

4. Composé cosmétique comprenant l'adsorbant de sébum décrit dans la revendication 1, 2 ou 3.

5. Procédé d'adsorption de sébum, le procédé comprenant l'application d'un oxyde de zinc sphérique d'oxyde de zinc lamellaire agrégé ayant une taille médiane de 0,01 µm ou plus mais de moins de 10 µm et traité en surface avec un organopolysiloxane et/ou le triéthoxycaprylylsilane de sorte que de 0,5 à 10 parties en poids de l'organopolysiloxane et/ou du triéthoxycaprylylsilane est collé par 100 parties en poids de l'oxyde de zinc ou une composition comprenant ledit oxyde de zinc sphérique traité en surface de l'oxyde de zinc lamellaire agrégé sur la peau.

6. Utilisation d'un oxyde de zinc sphérique d'oxyde de zinc lamellaire agrégé ayant une taille médiane de 0,01 µm ou plus mais de moins de 10 µm et traité en surface avec un organopolysiloxane et/ou le triéthoxycaprylylsilane de sorte que de 0,5 à 10 parties en poids de l'organopolysiloxane et/ou du triéthoxycaprylylsilane est collé par 100 parties en poids de l'oxyde de zinc ou d'une composition comprenant ledit oxyde de zinc traité en surface de l'oxyde de zinc lamellaire agrégé pour l'adsorption du sébum par application sur la peau.
